# EUROPEAN PATENT APPLICATION

(11) **EP 0 934 744 A1**
(43) Date of publication of application: **11.08.1999**
(21) Application number: 98301172.7
(22) Date of filing: 13.02.1998
(51) Int. Cl.: A61K 31/21

(54) **Nitroglycerin preparation for treatment of erectile dysfunction**

(30) Priority: 30.01.1998 GB 9802078
(71) Applicant: Futura Medical Limited, Petersfield, Hampshire GU31 4AD (GB)
(72) Inventor: Kemp, Colin Anthony, Hampshire P09 1AX (GB)
(74) Representative: Harrison, Ivor Stanley

(57) **Abstract**

Glyceryl trinitrate and lanolin in combination are used in the preparation of compositions for the treatment of erectile dysfunction in human males and for a method of cosmetic treatment of human males.

## Description

This invention relates to a preparation for topical application to the male sexual organ, especially for the treatment of erectile dysfunction.

Erectile dysfunction is a widespread problem with human males, albeit not commonly discussed in public. It is not regarded as a medical condition susceptible to treatment in the conventional sense being to a large extent caused by a mental condition or at least exacerbated by a loss of confidence following an initial experience caused perhaps by tiredness or an over-indulgence in food or alcohol. Nevertheless, the consequences are serious to the sufferer, resulting in psychological damage and in many cases frustrating the normal fertilisation processes so that artificial insemination procedures must be used if procreation is required. Procedures for overcoming this problem have been proposed which can involve the surgical implantation of stiffening devices as described in GB-A-1476804, US-A-5242391 and other patents. Chemotherapeutical approaches have been proposed which require the injection of vasodilator compositions immediately prior to intercourse. The injection takes place on or near the penile shaft. Neither procedure is attractive and both approaches can result in revulsion or distress on the part of the partners involved. US-A-5059603 and US-A-4801587 propose topical application of compositions containing vasodilators. US-A-5059603 proposes the use of a composition including a combination of a vasodilator and vasoconstrictor in a carrier intended to assist absorption of the active ingredients through the skin of the penis, the active ingredients being chosen so that the vasodilator acts more quickly than the vasoconstrictor whereby an erection of the penis, having been initiated by the action of the vasodilator, is sustained by the action of the vasoconstrictor. Vasodilators said to be appropriate for use in such compositions include nitroglycerin, papaverine, hydralazine, sodium nitroprusside and phenoxybenzamine, among others. Vasoconstrictors include caffeine, 3-(2-aminoethyl)indole derivatives and adrenaline. US-A-4801587 proposes a composition in the form of an ointment and including a vasolidator selected from papaverine, hydralazine, sodium nitroprusside, phenoxybenzamine and phentolamine mixed in a base with dimethylsulfoxide as a skin-absorption agent. Dimethylsulfonide is also proposed for the same purpose in US-A-5059603.

It has now been found that nitroglycerin (glyceryl trinitrate) can be used as a vasodilator in a composition for topical application to the penis in conjunction with carrier materials without either a vasoconstrictor or dimethyl sulfoxide, which is known to be a toxic material, and that such compositions are effective for the treatment of erectile dysfunction.

According to one aspect of the present invention, there is provided the use of a combination of compounds in the preparation of a composition for the treatment of erectile dysfunction in human males, the compounds comprising glyceryl trinitrate and lanolin.

According to another aspect of the invention, a method of treatment of erectile dysfunction in males comprises applying to the penis a composition comprising an effective amount of glyceryl trinitrate and lanolin, and causing or permitting the composition to penetrate the skin of the penile shaft.

The carrying our of the method of the invention before sexual intercourse results in an erection of the penis which not only enables subsequent intercourse to take place but also provides for the subject male a mental state of renewed confidence in his ability as a sexual partner and may provide for the other partner a sense of relief from any self-doubt concerning her abilities to stimulate her partner to a state of sexual arousal. The method according to the invention is thus a means of acquisition of a particular state of mental well-being or a method of enhancing or promoting sexual appeal or sexual confidence, with cosmetic overtones in terms of enhancing the appearance of the male anatomy in the circumstances of intimate sexual activity.

The invention also includes, in a further aspect, the use of glyceryl trinitrate and lanolin in the treatment of erectile dysfunction in human males.

The glyceryl trinitrate acts as a vasodilator according to its established utility for example in the treatment of angina pectoris but it has not hitherto been recognised that in simple combination with lanolin there could be provided an effective treatment of erectile dysfunction in human males.

Glyceryl trinitrate is a material which is per se explosive. For use in the present invention, it is preferably adsorbed on a solid stabilizer in finely-divided particulate form, the particles being in the sub-micron size range to render them capable of passage through the skin. A preferred stabilizer compound is lactose on which the glyceryl trinitrate may be adsorbed at a concentration of from 5 to 20% by weight, for example 10% by weight.

The lanolin acts as a lubricant or moisturiser and also enhances skin absorption. It is known to be non-toxic and for the purpose of the present invention should be used as a medical grade such as "Medilan" (Trade Mark).

Preferably, compositions according to the invention also include a physical stabilizer such as a paraffin cream; optionally, the compositions contain other ingredients such as fragrances and/or colorants. The paraffin cream, for example provided as white Soft Paraffin B.P., promotes stability of the composition on the skin and also acts as a water-repellant barrier and lubricant.

The composition also includes water to adjust the overall viscosity or consistency and concentration of the active ingredient to the desired levels, bearing in mind that for ease of use the composition should be in the form of a physically-stable suspension of the active ingredient in a creamy emulsion which is sufficiently low in viscosity to be readily applied to and spread over the skin of the penis at ordinary body temperatures.

An example of a composition for use according to the present invention is given below:

| Ingredient | Weight % |
|---|---|
| Glyceryl trinitrate (10% in lactose) | 10 |
| Lanolin ("Medilan") | 44 |
| White Soft Paraffin B.P. | 21 |
| Demineralized Water | 25 |

In use, two grams of the above formulation, which has the form of a white cream, was applied to the glans penis and penile shaft and gently massaged into the skin. The effect was to produce an erection of the penis which was sustained for approximately 45 minutes. The effect in producing an erection way be enhanced by other stimuli of tactile, audio and/or visual nature.

## Claims

1. Use of a combination of compounds in the preparation of a composition for the treatment of erectile dysfunction in human males, the compounds comprising glyceryl trinitrate and lanolin.

2. A method of cosmetic treatment for improving the bodily appearance of human males, the method comprising applying to the penis a composition comprising an effective amount of glyceryl trinitrate and lanolin, and causing or permitting the composition to penetrate the skin.

3. The use of glyceryl trinitrate and lanolin in combination as a cosmetic product.

4. The use as claimed in claim 1 or claim 3 or a method of cosmetic treatment as claimed in claim 2, in which the glyceryl trinitrate is adsorbed on a solid stabilizer in finely-divided particulate form.

5. The use or the method as claimed in claim 4, in which the stabilizer compound comprises lactose.

6. The use or the method according to claim 5, in which the glyceryl trinitrate is adsorbed on the lactose at a concentration of from 5 to 20% by weight.

7. The use or the method according to any preceding claim, in which the composition also includes a physical stabilizer.

8. The use or the method according to claim 7, in which the composition also includes water.

9. The use or the method according to any preceding claim, in which the composition consists essentially of 10wt% glyceryl trinitrate (10% in lactose), 44wt% lanolin, 21wt% white soft paraffin B.P. and 25wt% demineralized water.
